# FASCICULE DE BREVET EUROPEEN

(11) **EP 1 169 118 B1**
(45) Date de publication et mention de la délivrance du brevet: **06.11.2002**
(21) Numéro de dépôt: 00915284.4
(22) Date de dépôt: 05.04.2000
(51) Int. Cl.: B01J 2/16, B01J 8/24, B01J 8/32, B01J 2/00

(54) **PROCEDE D'ENROBAGE ET DE PELLICULAGE DE POUDRES FINES DANS UN ENROBEUR A LIT FLUIDISE**
VERFAHREN ZUR UMHÜLLUNG UND FILMBESCHICHTUNG FEINER PULVER MITTELS EINER WIRBELSCHICHTVORRICHTUNG
METHOD FOR COATING AND FILM-COATING FINE POWDERS IN A FLUIDISED BED COATING UNIT

(30) Priorité: 09.04.1999 FR 9904433
(43) Date de publication de la demande: 09.01.2002
(73) Titulaire: INSTITUT NATIONAL POLYTECHNIQUE DE TOULOUSE (I.N.P.T.), 31029 Toulouse Cédex 4 (FR)
(72) Inventeur: HEMATI, Mehrdji, F-31100 Toulouse (FR); SALEH, Khashayar, F-31300 Toulouse (FR); STEINMETZ, Daniel, F-31400 Toulouse (FR)
(74) Mandataire: Rinuy, Santarelli
(86) Numéro de dépôt international: FR0000841
(87) Numéro de publication internationale: WO00061274

(56) Documents cités:
- EP-A- 0 616 841
- WO-A-93/24215
- US-A- 4 379 186

## Description

L'invention concerne un procédé pour recouvrir par voie sèche de fines particules par une couche de produit solide inerte ou actif. Il s'agit des poudres de tailles de grains inférieures à 160 micromètres et de masses volumiques supérieures à 1000 kg/m3. Par ailleurs, cette technique permet aussi de déposer les éléments actifs à la surface interne des particules microporeuses. L'enrobage de fines particules, dans l'intention de les isoler, de les protéger contre leur environnement (humidité, lumière, oxygène...), de leur attribuer certaines propriétés surfaciques (hydrophobie, catalytique, écoulement) ou de les faire grossir, présente des enjeux considérables dans les industries de fabrication et de mise en forme des engrais chimiques, des détergents, des semences, des produits cosmétiques, dans le secteur agro-alimentaire et surtout dans l'industrie pharmaceutique, ainsi que dans toute industrie qui a à traiter ou à transformer des solides pulvérulents. Dans l'industrie pharmaceutique, les objectifs principaux de cette opération sont le masquage de goûts, l'amélioration de l'aspect du produit, et enfin le maintien du profil de dissolution in vitro de médicament. Par ailleurs, l'enrobage peut aussi être envisagé pour la fabrication des catalyseurs supportés.

On connaît classiquement des procédés de ce domaine, tels que en particulier l'enrobage de particules par voie sèche, qui fait appel souvent à la technologie des contacteurs à lit fluidisé, car celle-ci permet d'assurer un traitement uniforme et individuel des grains. Le principe de cette technique consiste à introduire dans un lit fluidisé des particules solides chaudes le produit enrobant (sous forme d'une solution ou d'une suspension dans un solvant facilement évaporable) par l'intermédiaire d'un système de pulvérisation adéquat. Ainsi, on peut réaliser les différentes opérations telles que le mélange, la pulvérisation, le mouillage, le dépôt et le séchage dans le même appareil. Cette technologie est bien adaptée au traitement des poudres ayant une taille moyenne de grains supérieure à 300 micromètres.

De façon connue, ces procédés présentent tous l'inconvénient, pour des tailles de grains inférieures à 300 micromètres, de conduire au phénomène de défluidisation et à la prise en masse du lit résultant d'une agglomération non contrôlée. Afin de contrôler l'opération et de minimiser la quantité d'agglomérats formés, les techniques actuellement employées reposent soit sur l'utilisation des débits importants du gaz de fluidisation (enrobeur à lit fluidisé circulant), soit sur l'emploi des enrobeurs munis d'agitateurs mécaniques. Ces différentes configurations posent des problèmes tels que l'attrition, la perte de produit actif, l'emploi des systèmes de dépoussiérage importants et les problèmes de conception et de manutention du solide.

L'invention vise quant-à-elle un procédé de mise en oeuvre simple, permettant de résoudre les problèmes pré-cités.

L'invention propose à cet effet un procédé d'enrobage et de pelliculage de poudres fines, caractérisé en ce que l'on ajoute aux fines particules une quantité adéquate de grosses particules (taille environ entre 0,4 et 1 mm) de l'ordre de 30%. Ces dernières peuvent avoir ou non les mêmes propriétés que celles des particules fines. L'opération est réalisée dans un pilote comportant l'enrobeur à lit fluidisé, le circuit de gaz et son préchauffage, le système de dépoussiérage du gaz sortant de l'enrobeur (cyclones et filtres), le circuit du liquide (réservoir de stockage, filtres, pompe, débitmètres), le système de pulvérisation de liquide (système bi-fluide, buse à mélange interne et/ou externe), le système de régulation de la température de l'enrobeur avec un capteur de température, et le système d'acquisition et traitement de données.

On comprend que cette disposition permet une mise en oeuvre avantageuse de l'invention, avec une simplicité technologique, une efficacité élevée et un faible coût énergétique.

La description et les dessins d'un mode préféré de réalisation de l'invention, donnés ci-après, permettront de mieux comprendre les buts et avantages de l'invention. Il est clair que cette description est donnée à titre d'exemple, et n'a pas de caractère limitatif. Dans les dessins :
- la figure 1 représente les différentes étapes du procédé,
- la figure 2 représente l'évolution du pourcentage massique d'agglomérats formés au cours de l'enrobage,
- la figure 3 représente l'évolution du taux de croissance et de la porosité des particules d'alumine poreuse au cours de l'opération.

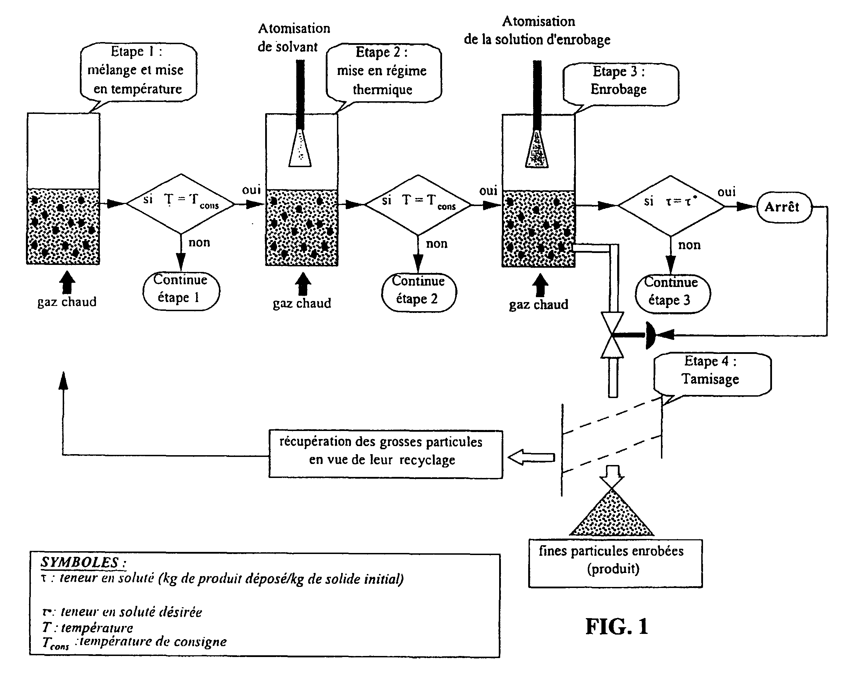

Comme on le voit sur la figure 1, un procédé selon l'invention comporte les étapes suivantes, avec les conditions opératoires précisées ci-dessous :

### Etape (1), mélange et mise en régime thermique :

1) les deux lots de particules solides (fines et grosses) sont fluidisés à l'aide d'un courant de gaz chaud,
2) la vitesse de fluidisation est fixée à environ deux fois la vitesse de fluidisation complète pour assurer un mélange intime des deux populations de particules solides,
3) cette opération continue jusqu'à ce que la température du milieu fluidisé atteigne la valeur de consigne,

### Etape (2), atomisation du solvant et mise en régime thermique :

4) une fois la température de consigne atteinte, on procède à la pulvérisation de solvant, avec le même débit que celui fixé lors de l'opération d'enrobage. La nature du solvant est identique à celle utilisée dans la préparation de la solution d'enrobage,
5) cette opération dure jusqu'à ce que la température du lit fluidisé atteigne celle de la consigne,

### Etape (3), enrobage proprement dit :

6) après cette période de mise en régime thermique, le solvant est remplacé par la solution d'enrobage,
7) cette étape dure jusqu'au moment où la quantité de solide déposé sur les fines particules atteint la valeur désirée,

### Etape (4), séparation :

8) à la fin de l'opération, la séparation entre les fines particules enrobées et les grosses particules est réalisée par un simple tamisage. Les grosses particules ainsi récupérées peuvent être recyclées dans un nouveau cycle d'enrobage.

On notera dans le procédé que :
- on ajoute aux fines particules une quantité adéquate de grosses particules (taille environ entre 0,4 et 1 mm) de l'ordre de 30%,
- les poudres ont une taille de grains supérieure à 10 micromètres et une masse volumique supérieure à 1000 kg/m3,
- les grosses particules sont non-poreuses,
- on utilise une solution enrobante constituée d'un sel inorganique ou des solutions contenant des produits organiques ayant une faible viscosité (inférieure à 50 centipoises),
- la solution est introduite à l'aide d'un système de pulvérisation à bi-fluide avec un rapport entre le débit d'air de pulvérisation et celui de la solution compris entre 1000 à 2000 m3 d'air / m3 de solution;
- la buse du système de pulvérisation est disposée à la surface du lit au repos,
- la fluidisation utilise un gaz dont la vitesse est supérieure à deux fois la vitesse complète de fluidisation du mélange initial,
- le procédé peut être adapté à une opération continue.

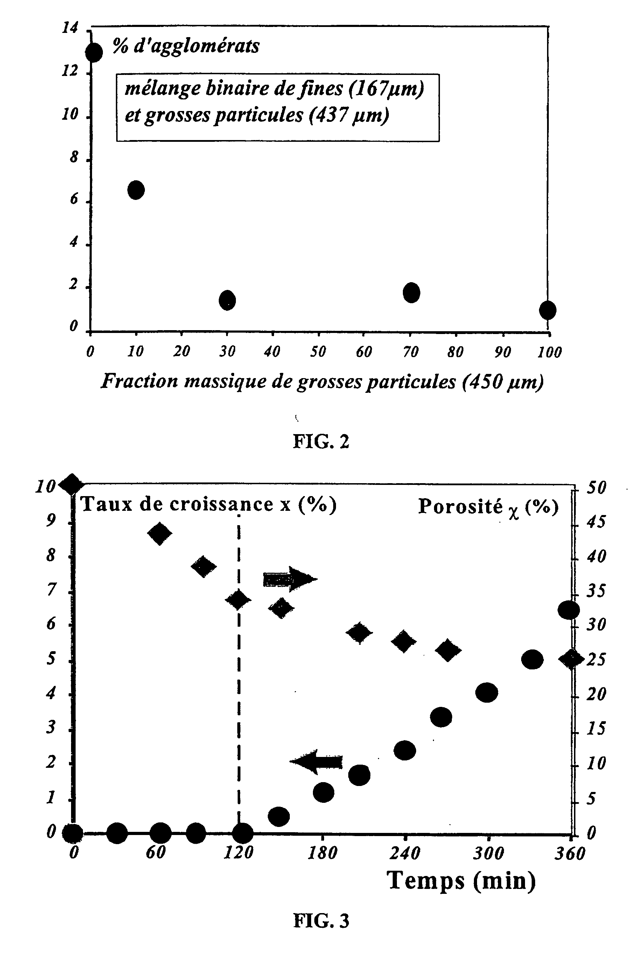

On comprend que l'invention permet d'empêcher la formation des agglomérats à l'échelle locale lors de la fluidisation, puisque la présence des grosses particules dans le lit joue le rôle d'un agitateur mécanique. Par ailleurs, ce nouveau procédé permet de réaliser l'enrobage avec un débit d'air de fluidisation raisonnable (environ quatre fois plus faible que celui utilisé dans les techniques traditionnelles), avec une efficacité d'opération élevée (supérieure à 95%). Il faut signaler que la mesure de la teneur en produit déposé sur les grosses particules a montré qu'elle reste extrêmement faible, autrement dit les grosses particules ne participent pas à l'enrobage. De plus, la séparation des grosses particules du mélange en fin d'opération peut se faire par simple tamisage. La figure 2 représente l'influence du pourcentage massique des grosses particules sur le taux d'agglomérats formés. La qualité du dépôt à la surface de particules non poreuses est fortement liée aux propriétés physico-chimiques de la solution enrobante et à celles du support, aux conditions de pulvérisation, à la nature du système de pulvérisation et à la position des buses par rapport au distributeur.

L'invention s'applique en particulier au domaine de l'enrobage de fines particules solides de masse volumique supérieure à 1000 kg/m3, et de taille de grains comprise entre 10 et 100 micromètres en utilisant des solutions contenant des sels minéraux. De plus, quelques essais préliminaires réalisés avec des solutions contenant des produits organiques (polymères) ont montré l'applicabilité du procédé selon l'invention.

Pour l'enrobage de fines particules microporeuses, l'évolution au cours de l'opération des propriétés texturales du produit (porosité, surface spécifique) a montré qu'initialement le dépôt est effectué à la surface interne des pores (période de non-croissance). Une fois que les sites actifs sont comblés, le dépôt peut s'effectuer à la surface externe du solide. La figure 3 présente l'évolution au cours du traitement du taux de croissance et de la porosité des particules d'alumine microporeuse. Ces résultats permettent d'envisager l'application du procédé au domaine de la fabrication de catalyseurs supportés. En effet, l'invention permet de réaliser les différentes étapes de la préparation des catalyseurs, l'imprégnation, le séchage et le mélange dans le même appareil. De plus, grâce au traitement uniforme de particules et à l'absence du phénomène d'agglomération, l'étape de broyage peut être éliminée. Par ailleurs, la dernière étape de la fabrication du catalyseur, qui consiste à calciner le produit, peut également être réalisée dans le même appareil.

De nombreuses variantes peuvent être considérées en fonction des circonstances d'utilisation, tant en ce qui concerne la nature des solutions d'enrobage, qui peuvent être soit des produits minéraux, mais également organiques, tels que des polymères, que la nature de l'opération, qui a été décrite précédemment en mode discontinu, mais qui peut également être réalisée en mode continu, et en fonction de l'application, qui concerne tant l'enrobage de solides pulvérulents que la fabrication de catalyseurs supportés.

La portée de la présente invention ne se limite pas aux détails des formes de réalisation ci-dessus considérées à titre d'exemple, mais s'étend au contraire aux modifications à la portée de l'homme de l'art.

## Revendications

1. Procédé d'enrobage et de pelliculage de poudres fines, dans lequel, par l'intermédiaire d'un système de pulvérisation adéquat, une solution enrobante ou une suspension dans un solvant facilement évaporable est introduite dans un lit fluidisé comportant de fines particules solides d'une taille comprise entre 10 et 160 micromètres, **caractérisé en ce que** l'on ajoute aux fines particules une quantité adéquate de grosses particules (taille environ entre 0,4 et 1 mm) de l'ordre de 30%.

2. Procédé selon la revendication 1 **caractérisé en ce que** les poudres ont une taille de grains inférieure à 100 micromètres et une masse volumique supérieure à 1000 kg/m3.

3. Procédé selon les revendications 1 et 2, **caractérisé en ce que** les grosses particules sont non-poreuses.

4. Procédé selon l'une des revendications 1 à 3, **caractérisé en ce qu'**on utilise une solution enrobante constituée d'un sel inorganique ou des solutions contenant des produits organiques ayant une faible viscosité (inférieure à 50 centipoises).

5. Procédé selon l'une des revendications 1 à 4, **caractérisé** en ce la solution est introduite à l'aide d'un système de pulvérisation à bi-fluide avec un rapport entre le débit d'air de pulvérisation et celui de la solution compris entre 1000 à 2000 m3 d'air / m3 de solution.

6. Procédé selon l'une des revendications 1 à 5, **caractérisé en ce que** la buse du système de pulvérisation est disposée à la surface du lit au repos.

7. Procédé selon l'une des revendications 1 à 6, **caractérisé en ce que** la fluidisation utilise un gaz dont la vitesse est supérieure à deux fois la vitesse complète de fluidisation du mélange initial.

8. Procédé selon l'une des revendications 1 à 7, **caractérisé en ce qu'**il peut être adapté à une opération continue.

## Claims

1. Method of coating and film-coating fine powders in which, by means of a suitable spraying system, a coating solution or a suspension in an easily evaporatable solvent is introduced into a fluidised bed comprising fine solid particles with a size between 10 and 160 micrometres, **characterised in that** a suitable quantity of coarse particles (size approximately between 0.4 and 1 mm) of around 30% is added to the fine particles.

2. Method according to Claim 1, **characterised in that** the particles have a grain size of less than 100 micrometres and a density greater than 1000 kg/m³.

3. Method according to Claims 1 and 2, **characterised in that** the coarse particles are non-porous.

4. Method according to one of Claims 1 to 3, **characterised in that** use is made of a coating solution consisting of an inorganic salt or solutions containing organic substances having a low viscosity (less than 50 centipoise).

5. Method according to one of Claims 1 to 4, **characterised in that** the solution is introduced by means of a dual-fluid spraying system with a ratio between the output of spraying air and that of the solution of between 1000 to 2000 m³ of air/m³ of solution.

6. Method according to one of Claims 1 to 5, **characterised in that** the nozzle of the spraying system is disposed on the surface of the bed at rest.

7. Method according to one of Claims 1 to 6, **characterised in that** the fluidisation uses a gas whose speed is greater than twice the complete fluidisation speed of the initial mixture.

8. Method according to one of Claims 1 to 7, **characterised in that** it can be adapted to a continuous operation.

## Patentansprüche

1. Verfahren zur Umhüllung und Filmbeschichtung feiner Pulver, bei dem über ein geeignetes Zerstäubungssystem eine umhüllende Lösung oder eine Suspension in einem leicht verdampfbaren Lösungsmittel in ein Wirbelbett eingeführt wird, das feine feste Teilchen mit einer Größe von 10 bis 160 Mikrometer enthält, **dadurch gekennzeichnet, daß** man den feinen Teilchen eine geeignete Menge großer Teilchen (Größe etwa 0,4 bis 1 mm) von etwa 30% zusetzt.

2. Verfahren nach Anspruch 1, **dadurch gekennzeichnet, daß** die Pulver eine Korngröße unter 100 Mikrometer und eine Dichte über 1000 kg/m³ haben.

3. Verfahren nach den Ansprüchen 1 und 2, **dadurch gekennzeichnet, daß** die großen Teilchen nicht-porös sind.

4. Verfahren nach einem der Ansprüche 1 bis 3, **dadurch gekennzeichnet, daß** man eine aus einem anorganischen Salz bestehende umhüllende Lösung oder Lösungen verwendet, die organische Produkte mit einer niedrigen Viskosität (unter 50 Zentipoise) enthalten.

5. Verfahren nach einem der Ansprüche 1 bis 4, **dadurch gekennzeichnet, daß** die Lösung mit Hilfe eines Zweifluid-Zerstäubungssystems mit einem Verhältnis zwischen dem Zerstäubungsluftdurchsatz und dem Durchsatz der Lösung von 1000 bis 2000 m³ Luft/m³ Lösung eingeführt wird.

6. Verfahren nach einem der Ansprüche 1 bis 5, **dadurch gekennzeichnet, daß** die Düse des Zerstäubungssystems an der Oberfläche des Betts im Ruhezustand angeordnet ist.

7. Verfahren nach einem der Ansprüche 1 bis 6, **dadurch gekennzeichnet, daß** die Wirbelschichtbildung ein Gas verwendet, dessen Geschwindigkeit größer als das Zweifache der vollständigen Fluidisierungsgeschwindigkeit der Anfangsmischung ist.

8. Verfahren nach einem der Ansprüche 1 bis 7, **dadurch gekennzeichnet, daß** es an einen kontinuierlichen Arbeitsgang adaptiert werden kann.
